# EUROPÄISCHE PATENTSCHRIFT

(11) **EP 4 215 158 B1**
(45) Veröffentlichungstag und Bekanntmachung des Hinweises auf die Patenterteilung: **06.11.2024**
(21) Anmeldenummer: 23152701.1
(22) Anmeldetag: 20.01.2023
(51) Int. Cl.: A61F 2/00, A61B 5/389, A61F 2/48

(54) **KÜNSTLICHER SCHLIESSMUSKEL**
ARTIFICIAL SPHINCTER
SPHINCTER ARTIFICIEL

(30) Priorität: 24.01.2022 DE 102022101549
(43) Veröffentlichungstag der Anmeldung: 26.07.2023
(73) Patentinhaber: Friedrich-Alexander-Universität Erlangen-Nürnberg, 91054 Erlangen (DE)
(72) Erfinder: BENKE, Elisabeth, 91052 Erlangen (DE); PREIS, Alexander, 91052 Erlangen (DE); REITELSHÖFER, Sebastian, 91207 Lauf (DE); RIEKER, Ralf, 91054 Erlangen (DE); EBERT, Thomas, 90513 Zirndorf (DE)
(74) Vertreter: Dr. Gassner & Partner mbB

(56) Entgegenhaltungen:
- WO-A2-2012/127420
- DE-A1- 102017 115 288

## Beschreibung

Die Erfindung betrifft einen künstlichen Schließmuskel, der in die Harnröhre eines Patienten einsetzbar ist, mit einem eine Durchgangsöffnung aufweisenden Gehäuse und einem in der Durchgangsöffnung angeordneten Schließmechanismus, der zwischen einer ersten Stellung, in der die Durchgangsöffnung freigegeben ist, und einer zweiten Stellung, in der die Durchgangsöffnung verschlossen ist, bewegbar ist, wobei der künstliche Schließmuskel wenigstens einen zum Erfassen eines körpereigenen Biosignals ausgebildeten Sensor aufweist oder mit einem derartigen Sensor verbunden ist und eine zum Auswerten des Biosignals ausgebildete Verarbeitungs- und Steuerungseinheit aufweist, durch die der Schließmechanismus in Abhängigkeit des ausgewerteten Biosignals steuerbar ist.

Ein derartiger künstlicher Schließmuskel in Form eines Implantats ist beispielsweise aus der WO 2012/127420 A2 bekannt. Dieses Implantat umfasst einen oder mehrere Drucksensoren, der oder die mit einem Sensormodul gekoppelt ist bzw. sind, so dass ein Ventil des Implantats anhand von Daten des Drucksensors oder der Drucksensoren steuerbar ist.

Ein künstlicher Schließmuskel wird auch in der DE 10 2017 115 288 A1 beschrieben.

Eine steigende Anzahl von Personen leidet an einer Form der Harninkontinenz. In der Altersgruppe der über 80-Jährigen steigt die Prävalenz auf nahezu 30%. Die Harninkontinenz kann zu einer erheblichen psychischen Belastung führen und beeinträchtigt die Lebensqualität des Patienten maßgeblich. Eine der häufigsten Formen der Harninkontinenz ist die sogenannte Stress- oder Belastungsinkontinenz. Bei Frauen tritt die Stressinkontinenz z. B. aufgrund der verminderten Beckenbodenintegrität nach Geburten auf, bei Männern zumeist nach chirurgischen Eingriffen an der Prostata. Bei einer leichteren Belastungsinkontinenz können zunächst konservative Maßnahmen vorgenommen werden, wie z. B. Beckenbodengymnastik, Magnetfeldbehandlung, Biofeedbacktraining, Elektrostimulationstraining oder Vibrationstherapie. In schwereren Fällen kommen jedoch nur operative Therapien in Frage. Die gängigsten Maßnahmen sind z. B. intraurethrale submuköse Injektionen von Füllstoffen, die Implantation eines Beckenbodennetzes oder eine paraurethrale Ballonkompression bei Männern. Diese herkömmlichen Therapieverfahren verhelfen den Patienten nicht immerzu einer vollständigen Kontinenz. Des Weiteren ist die Implantation eines künstlichen Schließmuskelsystems mit einem großen chirurgischen Eingriff verbunden, dies stellt ein erhöhtes Potenzial für Komplikationen nach der Operation da.

Bei einem in der Praxis verwendeten System wird der Schließmechanismus betätigt, indem man einen im Skrotum bzw. den Labia majora platzierten Pumpmechanismus betätigt oder einen teilweise rotierenden Permanentmagneten in die Nähe des im Körper implantierten künstlichen Schließmuskels bewegt. Eine derartige manuelle Bedienung ist allerdings wenig intuitiv und unter Umständen sehr indiskret. Eine fachgerechte Bedienung erfordert zudem motorisches Geschick.

Der Erfindung liegt daher die Aufgabe zugrunde, einen künstlichen Schließmuskel anzugeben, der einfacher bedient werden kann.

Zur Lösung dieser Aufgabe ist ein künstlicher Schließmuskel mit den Merkmalen des Anspruchs 1 vorgeshen. Der erfindungsgemäße Schließmuskel zeichnet sich dadurch aus, dass der Sensor aus der folgenden Gruppe von Sensoren ausgewählt ist: elektromyografischer Sensor, elektroneurografischer Sensor, Körperschallsensor, Inertialsensor.

Die Erfindung beruht auf der Erkenntnis, dass eine unauffällige und diskrete Steuerung des in einem menschlichen Körper implantierten künstlichen Schließmuskels durch die Nutzung körpereigener Biosignale möglich ist, die durch den Sensor erfasst und an die Verarbeitungs- und Steuerungseinheit weitergeleitet werden. Darüber hinaus kann der erfindungsgemäße künstliche Schließmuskel intuitiv bedient werden. Der Benutzer kann die dazu erforderlichen Kommandos und Bedienvorgänge, die die Biosignale erzeugen, vergleichsweise einfach erlernen.

Bei dem erfindungsgemäßen künstlichen Schließmuskel ist gemäß einer Variante vorgesehen, dass der Sensor als elektromyografischer Sensor oder elektroneurografischer Sensor ausgebildet ist. Ein elektromyografischer Sensor ist in der Lage, die elektrische Muskelaktivität zu erfassen. Dementsprechend kann der Benutzer durch eine bewusste Muskelaktivität, zum Beispiel eine Muskelkontraktion, ein körpereigenes Biosignal erzeugen, das von dem Sensor erfasst werden kann. Nach der Auswertung des Biosignals kann der Schließmechanismus gesteuert werden, d. h. er kann geöffnet oder geschlossen werden. Es wird daher besonders bevorzugt, dass der Sensor zum Erfassen von Muskelkontraktionen des Patienten ausgebildet ist.

In ähnlicher Weise ist ein elektromyografischer Sensor in der Lage, die elektrische Funktion von Nerven zu erfassen. Eine Aktivität des Benutzers, beispielsweise eine bewusste Bewegung oder eine Muskelbetätigung, kann ein körpereigenes Biosignal erzeugen, das von dem elektromyografischen Sensor erfasst und verwendet wird, um den Schließmechanismus zu steuern.

Eine weitere Variante des erfindungsgemäßen künstlichen Schließmuskels sieht vor, dass der Sensor als Beschleunigungssensor, insbesondere als Körperschallsensor ausgebildet ist. Auf diese Weise können von dem Benutzer erzeugte Schallsignale erfasst werden, um den als Implantat ausgebildeten künstlichen Schließmuskel zu steuern. Beispielsweise können Schallsignale durch Klopfen erzeugt werden. Wenn der Benutzer auf einen bestimmten Bereich seines Körpers klopft, werden die dadurch erzeugten Schallsignale von dem Körperschallsensor erfasst, in der Verarbeitungs- und Steuerungseinheit ausgewertet und für die Steuerung des künstlichen Schließmuskels verwendet. Die Steuerung erfolgt dabei aktiv durch den Benutzer, anders als bei automatisch arbeitenden Vorrichtungen, bei denen der künstliche Schließmuskel automatisch betätigt wird.

Eine nicht erfindungsgemäße Variante des künstlichen Schließmuskels sieht vor, dass der Sensor zum Erfassen einer Druckdifferenz zwischen dem proximalen Ende und dem distalen Ende des Gehäuses ausgebildet ist. Alternativ kann der Sensor zwei Drucksensoren zum Erfassen von Absolutdrücken umfassen. Die Druckdifferenz kann dann einfach durch Bilden der Differenz der beiden erfassten Absolutdrücke ermittelt werden. Eine derartige Druckdifferenz kann beispielsweise durch eine gezielte Muskelkontraktion des Benutzers ausgelöst werden. Die Druckdifferenz ist ein Biosignal, das durch den Sensor erfasst und nach der Auswertung zu Steuerung des künstlichen Schließmuskels verwendet werden kann. Die Auswertung beruht darauf, dass eine Muskelkontraktion ein bestimmtes, charakteristisches oder einzigartiges Muster im Druckverlauf erzeugt. Eine bewusste Muskelkontraktion kann daher von anderen Bewegungen des Benutzers unterschieden werden.

Eine weitere alternative Variante des erfindungsgemäßen künstlichen Schließmuskels sieht vor, dass der Sensor als Bewegungssensor ausgebildet ist. Ein Bewegungssensor kann Bewegungen oder Beschleunigungen erfassen. Ein derartiges Biosignal kann zum Beispiel von dem Benutzer durch Klopfen auf einen bestimmten Körperbereich erzeugt werden. Dadurch wird ein charakteristisches Biosignal erzeugt, das anschließend zur Steuerung des künstlichen Schließmuskels dient. Die Verarbeitungs- und Steuerungseinheit ist in der Lage, ein bewusst erzeugtes, charakteristisches Beschleunigungssignal von einem anderen Beschleunigungssignal zu unterscheiden. Der implantierte künstliche Schließmuskel kann daher von seinem Benutzer mit hoher Präzision bedient werden.

Die Erfindung wird nachfolgend anhand von Ausführungsbeispielen unter Bezugnahme auf die Zeichnungen erläutert. Die Zeichnungen sind schematische Darstellungen und zeigen:
- Fig. 1: ein erstes Ausführungsbeispiel eines erfindungsgemäßen künstlichen Schließmuskels in einer geschnittenen Ansicht;
- Fig. 2: ein zweites Ausführungsbeispiel eines erfindungsgemäßen künstlichen Schließmuskels in einer geschnittenen Ansicht;
- Fig. 3: ein drittes Ausführungsbeispiel eines nicht erfindungsgemäßen künstlichen Schließmuskels in einer geschnittenen Ansicht;
- Fig. 4: ein viertes Ausführungsbeispiel eines erfindungsgemäßen künstlichen Schließmuskels in einer geschnittenen Ansicht;
- Fig. 5: ein Flussdiagramm, das die wesentlichen Schritte beim Betrieb des erfindungsgemäßen künstlichen Schließmuskels zeigt.

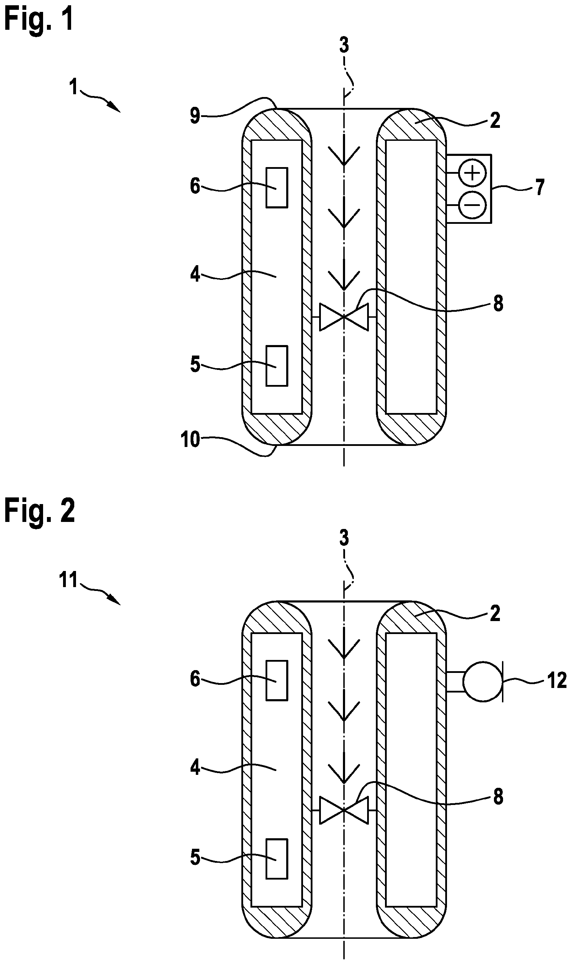

Der in Fig. 1 in einer geschnittenen Ansicht gezeigte künstliche Schließmuskel 1 ist als Implantat ausgebildet und umfasst ein als Hohlzylinder ausgebildetes Gehäuse 2, das eine zentrale Durchgangsöffnung 3 besitzt. Das Implantat ist operativ in die Harnröhre eines Patienten eingesetzt und befestigt worden. Die Durchgangsöffnung 3 weist eine schematisch dargestellte Aufnahme 4 auf, in der die technischen Komponenten untergebracht sind. Dazu zählen ein Energiespeicher 5, der diesem Ausführungsbeispiel als wiederaufladbare Batterie ausgebildet ist und eine Verarbeitungs- und Steuerungseinheit 6. An der Außenseite des Gehäuses 2 ist ein schematisch dargestellter Sensor angeordnet, der in diesem Ausführungsbeispiel als elektromyografischer Sensor 7 ausgebildet ist. In der Durchgangsöffnung 3 befindet sich ein Schließmechanismus in Form eines steuerbaren Ventils 8. Das Ventil 8 ist zwischen einer ersten Stellung, in der die Durchgangsöffnung 3 freigegeben ist, und einer zweiten Stellung, in der die Durchgangsöffnung 3 verschlossen ist, bewegbar. Wenn die Harnblase des inkontinenten Patienten gefüllt ist, befindet sich in dem Bereich oberhalb des Ventils 8 Harn. Der Benutzer kann dann den Schließmechanismus betätigen, um das Ventil 8 zu öffnen, damit Harn von dem proximalen Ende 9 durch die Durchgangsöffnung 3 zum distalen Ende 10 des künstlichen Schließmuskels 1 und weiter durch die Harnröhre abfließen kann.

Die Bedienung des künstlichen Schließmuskels 1 erfolgt durch eine von dem Patienten bewusst veranlasste Muskelkontraktion in der Nähe des künstlichen Schließmuskels 1, wodurch ein Biosignal erzeugt wird, das von dem elektromyografischen Sensor erfasst wird. Dieses Signal gelangt zu der Verarbeitungs- und Steuerungseinheit 6, die es auswertet und anhand des ausgewerteten Biosignals das Ventil 8 steuert, d. h. dieses öffnet oder schließt.

Fig. 2 zeigt ein weiteres Ausführungsbeispiel eines künstlichen Schließmuskels 11, der ähnlich wie der in Fig. 1 gezeigte künstliche Schließmuskel 1 aufgebaut ist. Auf eine detaillierte Beschreibung der übereinstimmenden Komponenten wird daher an dieser Stelle verzichtet. Der künstliche Schließmuskel 11 weist einen Beschleunigungssensor auf, der als Körperschallsensor 12 ausgebildet ist. Wenn der Benutzer gezielt ein bestimmtes Körperschallsignal erzeugt, beispielsweise durch Klopfen auf einen bestimmten Körperbereich, kann der erzeugte Körperschall mittels des Körperschallsensors 12 erfasst werden. Nach der Auswertung dieses Biosignale in der Verarbeitungs- und Steuerungseinheit 6 wird das Ventil 8 geöffnet bzw. geschlossen.

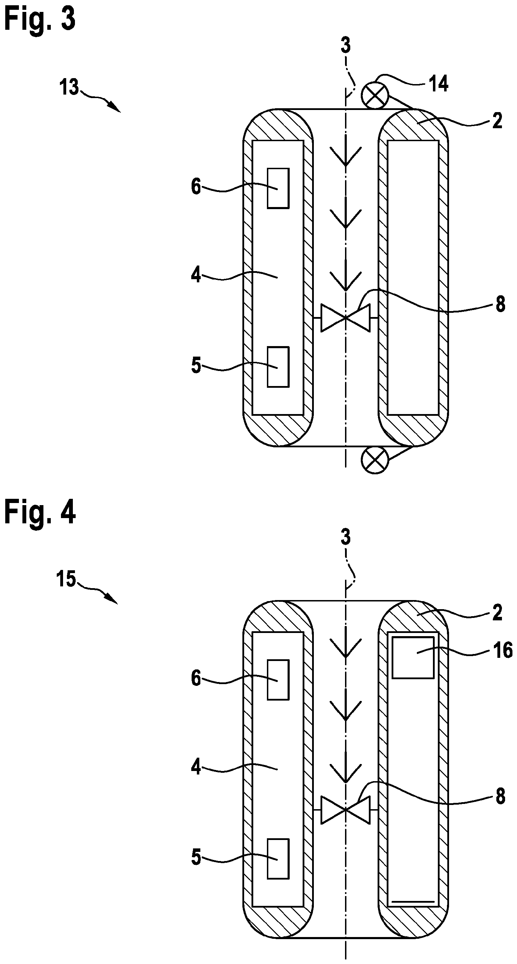

Fig. 3 zeigt ein weiteres Ausführungsbeispiel eines künstlichen Schließmuskels 13, der wiederum ähnlich wie der in Fig. 1 gezeigte künstliche Schließmuskel 1 aufgebaut ist. Der Sensor ist in diesem Ausführungsbeispiel als Drucksensor 14 ausgebildet und erfasst den im Bereich des künstlichen Schließmuskels 13 herrschenden Druck. Wenn der Benutzer das Ventil 8 des künstlichen Schließmuskels 13 betätigen will, erzeugt er eine Druckänderung, beispielsweise durch eine Muskelkontraktion oder durch eine Berührung eines bestimmten Körperbereichs in der Nähe des implantierten Schließmuskels 13. Die Druckänderung wird von dem Drucksensor 14 registriert, als Biosignal an die Verarbeitungs- und Steuerungseinheit 6 übermittelt und dort gefiltert und analysiert. In Abhängigkeit des Analyseergebnisses wird das Ventil 8 wie vom Benutzer gewünscht gesteuert, d. h. geöffnet oder geschlossen. Der Drucksensor ist in diesem Ausführungsbeispiel als Differenzdrucksensor ausgebildet. Alternativ können zwei separate Drucksensoren zum Erfassen von Absolutdrücken verwendet werden. Durch Differenzbildung der Druckwerte kann daraus die Druckdifferenz berechnet werden.

Fig. 4 zeigt ein weiteres Ausführungsbeispiel eines künstlichen Schließmuskels 15, der wiederum ähnlich wie der in Fig. 1 gezeigte künstliche Schließmuskel 1 aufgebaut ist. Der Sensor ist in diesem Ausführungsbeispiel als Inertialsensor 16 ausgebildet. Der Inertialsensor 16 umfasst eine Inertialmesseinheit (IMU) mit Beschleunigungssensoren und Drehratensensoren, die in der Lage ist, Bewegungen und Beschleunigungen zu erfassen. Durch Auswerten dieser Biosignale kann festgestellt werden, ob es sich um ein Bedienkommando des Benutzers oder ein anderes, nicht zu beachtendes Signal handelt. Wenn festgestellt wurde, dass es sich um ein Bedienkommando handelt, wird das Ventil 8 durch die Verarbeitungs- und Steuerungseinheit 6 betätigt, d. h. geöffnet oder geschlossen.

Die beschriebenen Sensoren sind lediglich als Beispiele zu verstehen. Neben den beschriebenen Typen von Sensoren können auch andere Sensoren verwendet werden. Es ist auch möglich, dass der künstliche Schließmuskel eine Kombination mehrerer unterschiedlicher Sensoren umfasst, die unterschiedliche Biosignale erfassen können.

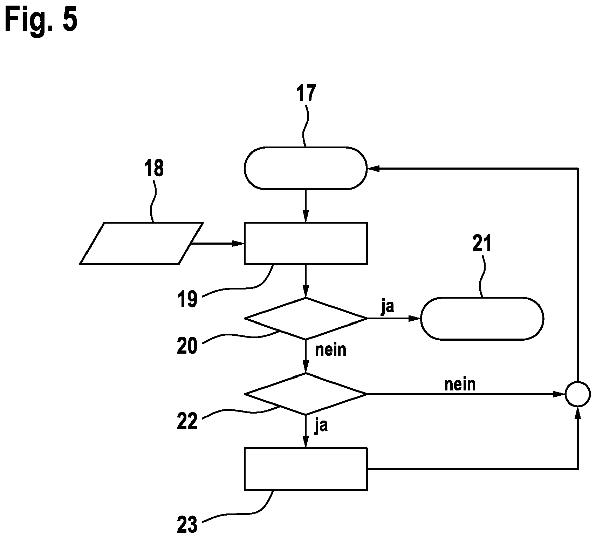

Fig. 5 ist ein Flussdiagramm, das die wesentlichen Schritte beim Betrieb des künstlichen Schließmuskels zeigt. Nach dem Start des Verfahrens im Schritt 17 werden Biosignale 18, die von dem Sensor bereitgestellt werden, im Schritt 19 gefiltert und analysiert. Im Schritt 20 wird anschließend von der Verarbeitungs- und Steuerungseinheit die Entscheidung getroffen, ob eine Deaktivierung des künstlichen Schließmuskels erforderlich ist. In bestimmten "Notfallsituationen" wird der künstliche Schließmuskel abgeschaltet oder die Durchgangsöffnung durch den durch den Schließmechanismus geöffnet. Ein Beispiel dafür ist ein stark erhöhter intravesikaler Druck, wenn die Durchgangsöffnung durch den Schließmechanismus geschlossen ist. In diesem Fall erfolgt eine selbstständige Deaktivierung und somit eine Öffnung der Durchgangsöffnung, um eine Beeinträchtigung des Patienten zu vermeiden.

Wenn im Schritt 20 entschieden wurde, dass eine Deaktivierung des künstlichen Schließmuskels erforderlich ist, wird im Schritt 21 der Betrieb des künstlichen Schließmuskels durch die Verarbeitungs- und Steuerungseinheit gestoppt. Eine Deaktivierung kann auch durch ein externes Signal erfolgen, beispielsweise, um eine Explanation oder eine Wartung des künstlichen Schließmuskels vorzunehmen. Andernfalls, wenn keine Deaktivierung erforderlich ist, wird im Schritt 22 geprüft, ob der Zustand des Ventils 8 geändert werden soll. Falls das Ergebnis dieser Entscheidung "ja" ist, wird im Schritt 23 die von dem Benutzer gewünschte Zustandsänderung vorgenommen. Wenn sich das Ventil in einem geöffneten Zustand befindet, wird es daraufhin geschlossen, wenn sich das Ventil 8 in einem geschlossenen Zustand befindet, wird es geöffnet. Andernfalls, wenn das Ergebnis der Entscheidung in dem Schritt 22 ist, dass keine Zustandsänderung erforderlich ist, wird das Verfahren im Schritt 19 fortgesetzt und in einer Schleife erneut durchgeführt.

### Bezugszeichenliste

- 1: künstlicher Schließmuskel
- 2: Gehäuse
- 3: Durchgangsöffnung
- 4: Aufnahme
- 5: Energiespeicher
- 6: Verarbeitungs- und Steuerungseinheit
- 7: elektromyografischer Sensor
- 8: Ventil
- 9: proximales Ende
- 10: distales Ende
- 11: künstlicher Schließmuskel
- 12: Körperschallsensor
- 13: künstlicher Schließmuskel
- 14: Drucksensor
- 15: künstlicher Schließmuskel
- 16: Inertialsensor
- 17: Schritt
- 18: Biosignal
- 19: Schritt
- 20: Schritt
- 21: Schritt
- 22: Schritt
- 23: Schritt

## Patentansprüche

1. Künstlicher Schließmuskel (1, 11, 15), der in die Harnröhre eines Patienten einsetzbar ist, mit einem eine Durchgangsöffnung (3) aufweisenden Gehäuse (2) und einem in der Durchgangsöffnung (3) angeordneten Schließmechanismus, der zwischen einer ersten Stellung, in der die Durchgangsöffnung (3) freigegeben ist, und einer zweiten Stellung, in der die Durchgangsöffnung (3) verschlossen ist, bewegbar ist, wobei der künstliche Schließmuskel (1, 11, 15) wenigstens einen zum Erfassen eines körpereigenen Biosignals ausgebildeten Sensor (7, 12, 16) aufweist oder mit einem derartigen Sensor (7, 12, 16) verbunden ist und eine zum Auswerten des Biosignals ausgebildete Verarbeitungs- und Steuerungseinheit (6) aufweist, durch die der Schließmechanismus in Abhängigkeit des ausgewerteten Biosignals steuerbar ist, **dadurch gekennzeichnet, dass** der Sensor (7, 12, 16) aus der folgenden Gruppe ausgewählt ist:
- elektromyografischer Sensor (7),
- elektroneurografischer Sensor,
- Körperschallsensor (12),
- Inertialsensor (16).

2. Künstlicher Schließmuskel nach Anspruch 1, wobei der elektromyografische Sensor (7) zum Erfassen von Muskelkontraktionen des Patienten ausgebildet ist.

3. Künstlicher Schließmuskel nach Anspruch 1, wobei der Körperschallsensor (12) zum Erfassen von durch den Patienten erzeugten Schallsignalen ausgebildet ist.

4. Künstlicher Schließmuskel nach Anspruch 3, wobei der Körperschallsensor (12) zum Erfassen von durch den Patienten erzeugten Klopfgeräuschen ausgebildet ist.

5. Künstlicher Schließmuskel nach Anspruch 1, wobei der Inertialsensor (16) zum Erfassen von durch den Patienten erzeugten Klopfgeräuschen ausgebildet ist.

## Claims

1. Artificial sphincter (1, 11, 15) insertable into the urethra of a patient, comprising a housing (2) having a passage opening (3) and a closing mechanism arranged in the passage opening (3) and movable between a first position in which the passage opening (3) is opened and a second position in which the passage opening (3) is closed, wherein the artificial sphincter (1, 11, 15) has at least one sensor (7, 12, 16) designed to detect an endogenous biosignal or is connected to such a sensor (7, 12, 16) and has a processing and control unit (6) designed to evaluate the biosignal, by means of which the closing mechanism can be controlled as a function of the evaluated biosignal, **characterized in that** the sensor (7, 12, 16) is selected from the following group:
- electromyographic sensor (7),
- electroneurographic sensor,
- structure-borne sound sensor (12),
- inertial sensor (16).

2. Artificial sphincter according to claim 1, wherein the electromyographic sensor (7) is designed to detect muscle contractions of the patient.

3. Artificial sphincter according to claim 1, wherein the structure-borne sound sensor (12) is designed to detect sound signals generated by the patient.

4. Artificial sphincter according to claim 3, wherein the structure-borne sound sensor (12) is designed to detect tapping noises generated by the patient.

5. Artificial sphincter according to claim 1, wherein the inertial sensor (16) is designed to detect tapping noises generated by the patient.

## Revendications

1. Sphincter artificiel (1, 11, 15) pouvant être inséré dans l'urètre d'un patient, ledit sphincter comporte un boîtier (2) présentant une ouverture de passage (3) et un mécanisme de fermeture disposé dans l'ouverture de passage (3), ledit mécanisme de fermeture pouvant se déplacer entre une première position dans laquelle l'ouverture de passage (3) est dégagée, et une deuxième position dans laquelle l'ouverture de passage (3) est fermée, en ce que le sphincter artificiel (1, 11, 15) comporte au moins un capteur (7, 12, 16) conçu pour la détection d'un biosignal de l'organisme ou est relié à un tel capteur (7, 12, 16) et comporte une unité de traitement et de commande (6) conçue pour l'évaluation du biosignal, par l'intermédiaire de laquelle le mécanisme de fermeture peut être commandé en fonction du biosignal évalué, **caractérisé en ce que** le capteur (7, 12, 16) est sélectionné dans le groupe suivant :
- capteur électromyographique (7)
- capteur électroneurographique,
- capteur de sons corporels (12)
- capteur inertiel (16).

2. Sphincter artificiel selon la revendication 1, en ce que le capteur électromyographique (7) est conçu pour la détection de contractions musculaires du patient.

3. Sphincter artificiel selon la revendication 1, en ce que le capteur de sons corporels (12) est conçu pour détecter des signaux sonores générés par le patient.

4. Sphincter artificiel selon la revendication 3, en ce que le capteur de sons corporels (12) est conçu pour détecter des bruits de battement générés par le patient.

5. Sphincter artificiel selon la revendication 1, en ce que le capteur inertiel (16) est conçu pour détecter des bruits de battement générés par le patient.
